# EUROPÄISCHE PATENTSCHRIFT

(11) **EP 1 301 163 B1**
(45) Veröffentlichungstag und Bekanntmachung des Hinweises auf die Patenterteilung: **26.01.2005**
(21) Anmeldenummer: 01945346.3
(22) Anmeldetag: 07.07.2001
(51) Int. Cl.: A61K 7/13

(54) **OXIDATIONSHAARFÄRBEMITTEL DIE ALS KUPPLERKOMPONENTE WENIGSTENS EINEN DISUBSTITUIERTEN 2,4-DIAMINOPHENYLETHER ENTHALTEN**
OXIDATIVE HAIR COLOURING AGENTS CONTAINING AT LEAST ONE DISUBSTITUITED 2,4-DIAMINO PHENYL ETHER AS A COUPLING COMPONENT
COLORANTS D'OXYDATION CAPILLAIRES RENFERMANT COMME COUPLEUR AU MOINS UN 2,4-DIAMINOPHENYLETHER DISUBSTITUE

(30) Priorität: 17.07.2000 DE 10034618
(43) Veröffentlichungstag der Anmeldung: 16.04.2003
(73) Patentinhaber: Henkel Kommanditgesellschaft auf Aktien, 40589 Düsseldorf (DE)
(72) Erfinder: ROSE, David, 40723 Hilden (DE); MEINIGKE, Bernd, 51381 Leverkusen (DE); HÖFFKES, Horst, 40595 Düsseldorf (DE)
(86) Internationale Anmeldenummer: PCT/EP2001/007827
(87) Internationale Veröffentlichungsnummer: WO 2002/005767

(56) Entgegenhaltungen:
- DE-A- 2 628 999
- DE-A- 19 826 457
- GB-A- 802 554
- US-A- 3 184 387
- US-A- 5 752 983
- US-A- 5 990 355
- US-A- 6 024 769
- G. O. GAEBEL ET AL : "Über Producte der partiellen und totalen Reduction des 2,6-Dinitrothymoläthyläthers" CHEMISCHE BERICHTE., Bd. 35, 1902, Seiten 2792-2802, XP002183576 VERLAG CHEMIE GMBH. WEINHEIM., DE ISSN: 0009-2940

## Beschreibung

Diese Erfindung betrifft Oxidationshaarfärbemittel, die in einem wässrigen Träger wenigstens eine Entwicklerkomponente und als Kupplerkomponente wenigstens einen in der Position 3 und 6 substituierten 2,4-Diaminophenylether enthalten.

Für das Färben von Keratinfasern, insbesondere von Haaren, spielen die sogenannten Oxidationsfärbemittel wegen ihrer intensiven Farben und guten Echtheitseigenschaften, die bei relativ niedriger Färbetemperatur und in kurzen Färbezeiten erzielt werden, eine besondere Rolle. Solche Färbemittel enthalten in einem geeigneten, meist wäßrigen Träger eine Oxidationsbase, die auch als Entwicklerkomponente bezeichnet wird, und die unter dem Einfluß von Luftsauerstoff oder von Oxidationsmitteln durch oxidative Polymerisation einen Farbstoff ausbildet. Dieser Farbstoff kann durch Kupplung mit einer anderen Entwicklerverbindung oder mit sogenannten Kupplerverbindungen, die selbst keine Farbstoffe ausbilden können, intensiviert und in der Nuance modifiziert werden.

Gute Oxidationsfarbstoffvorprodukte sollen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme, Reibung und den Einfluß chemischer Reduktionsmittel, z. B. Dauerwellenflüssigkeiten.

Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein. Weiterhin soll die erzielte Färbung durch Blondierung leicht wieder aus dem Haar entfernt werden können, falls sie doch nicht den individuellen Wünschen der einzelnen Person entspricht und rückgängig gemacht werden soll.

Als Entwicklerkomponenten werden üblicherweise primäre aromatische Amine mit einer weiteren, in para- oder ortho-Position befindlichen, freien oder substituierten Hydroxyoder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Beispiele solcher Entwickler sind z. B. p-Phenylendiamin, p-Toluylendiamin, 2-(2',5'-Diaminophenyl)-ethanol, 2-(2',5'-Diaminophenoxy)-ethanol, N,N-Bis-(2'hydroxyethyl)-p-phenylendiamin, N,N'-Bis-(4'-aminophenyl)-1,4-diazacycloheptan, 1,3-N-N'-Bis-(2'-hydroxyethyl)-N-N'-bis-(4'-aminophenyl)-diaminopropan-2-ol, 1,3-Bis-(2',5'-diaminophenoxy)-propan, p-Aminophenol, 4-Amino-3-methylphenol, 2-Aminoethyl-4-aminophenol, Bis-(2-hydroxy-5-aminophenyl)-methan, 1-(2'-Hydroxy-5'aminobenzyl)-imidazolin-2-on, 1-Phenyl-3-carboxanido-4-aminopyrazolon, 2,4,5,6-Tetraaminopyrimidin, 2-Hydroxy-4,5,6-triaminopyrimidin, 2,5,6-Triamino-4-hydroxypyrimidin, 2,4-Dihydroxy-5,6-diaminopyrimidin.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere 1-Naphtol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 1-Phenyl-3-methyl-pyrazolon-5, 2,4-Dichlor-3-aminophenol, 1,3-Bis-(2',4'-diaminophenoxy)-propan, 2-Chlor-resorcin, 2-Chlor-6-methyl-3-aminophenol, 2-Methylresorcin, 5-Methylresorcin, 2-Methyl-4-chlor-5-aminophenol und 2-Amino-3-hydroxypyridin.

Die Kuppler vom Typ der 2,4-Diaminophenylether wie z. B. das 2,4-Diaminoanisol oder das 2,4-Diaminophenoxyethanol liefern mit Entwicklern vom p-Phenylendiamin-Typus blaue Färbenuancen. Aus DE 26 28 999 A1 war ein in 6-Position alkylsubstituierter 2,4-Diaminoalkylether bekannt, der mit den bekannten Entwicklern vom p-Phenylendiamin-Typus braune bis gelbe Färbungen liefert, Leider weisen diese Kuppler den Nachteil auf, daß die Farbeniwicklung zu langsam verläuft, was bei der Anwendung zur Nachdunkelung des Farbtons noch nach Stunden führt.

Es sind überhaupt nur wenige Rotkuppler für p-Phenylendiamin-Derivate bekannt. Das Auffinden eines neuen Kupplers, der mit p-Phenylendiaminen intensive rote und braune Nuancen liefert, ist daher eine wertvolle Ergänzung der Palette der Oxidationsfarbstoffvorprodukte.

Gegenstand der Erfindung sind Oxidationsfärbemittel zum Färben von Keratinfasern, insbesondere von Haaren, die in einem wässrigen Träger wenigstens eine Entwicklerkomponente und als Kupplerkomponente wenigstens einen 2,4-Diaminophenylether der Formel (I), 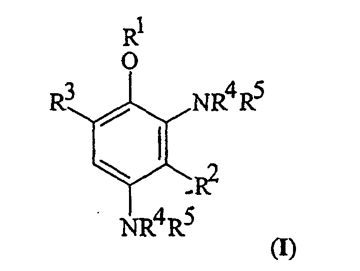 in der
- R¹ steht für eine C₁-C₄-Alkylgruppe, eine C₁-C₄-Monohydroxyalkylgruppe, eine C₂-C₄-Polyhydroxyalkylgruppe oder eine Phenylgruppe,
- R² und R³ unabhängig voneinander stehen für eine C₁-C₄-Alkylgruppe, eine C₁-C₄-Monohydroxyalkylgruppe oder eine C₂-C₄-Polyhydroxyalkylgruppe und
- R⁴ und R⁵ unabhängig voneinander stehen für Wasserstoff, eine C₁-C₄-Alkylgruppe, eine C₂-C₄-Monohydroxyalkylgruppe, eine C₂-C₄-Polyhydroxyalkylgruppe, eine Allylgruppe oder eine Benzylgruppe,
oder deren Salze enthalten.

Beispiele für die als Substituenten im Rahmen dieser Anmeldung genannten C₁-C₄-Alkylreste sind die Gruppen Methyl, Ethyl, Propyl, Isopropyl, Butyl, Isobutyl und tert.-Butyl. Ethyl und Methyl sind bevorzugte Alkylreste. Beispiele für bevorzugte C₂-C₄-Alkenylreste sind Vinyl und Allyl. Erfindungsgemäß bevorzugte C₁-C₄-Alkoxyreste sind beispielsweise eine Methoxy- oder eine Ethoxygruppe. Weiterhin können als bevorzugte Beispiele für eine C₁-C₄-Monohydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Beispiele für eine bevorzugte C₂-C₄-Polyhydroxyalkylgruppe sind die α,β-Dihydroxyethylgruppe oder die 2,3-Dihydroxypropylgruppe. Beispiele für Halogenatome sind erfindungsgemäß F-, Cl-, Br oder I-Atome, Cl-Atome sind ganz besonders bevorzugt. Die weiteren verwendeten Begriffe leiten sich erfindungsgemäß von den hier gegebenen Definitionen ab. Die in diesem Abschnitt getroffenen Definitionen gelten auch für die Formeln (E1), (E2), (E3), (E4) und (IIa) sowie (IIb).

In einer speziellen Ausführungsform enthalten die erfindungsgemäßen Oxidationsfärbemittel neben wenigstens einer Entwicklerkomponente wenigstens eine Verbindung gemäß Formel (I) oder deren Salz als Kupplerkomponente, wobei R¹ steht für eine C₁-C₄-Alkyl-oder eine C₂-C₄-Monohydroxyalkylgruppe.

Es ist bevorzugt, wenn die erfindungegemäßen Oxidationsfärbemittel neben wenigstens einer Entwicklerkomponente wenigstens eine Verbindung gemäß Formel (I) oder deren Salz als Kupplerkomponente enthalten, wobei die Reste R² und R³ stehen unabhängig voneinander für eine C₁-C₄-Alkyl- oder Monohydroxyalkylgruppe.

Weiterhin ist es bevorzugt, wenn die erfindungegemäßen Oxidationsfärbemittel neben wenigstens einer Entwicklerkomponente wenigstens eine Verbindung gemäß Formel (I) oder deren Salz als Kupplerkomponente enthalten, wobei die Reste R⁴ und R⁵ stehen für Wasserstoff.

Besonders bevorzugt ist es erfindungsgemäß, wenn das Oxidationsfärbemittel als Verbindung gemäß Formel (I) 2,4-Diamino-3,6-dimethylanisol enthält.

Die 2,4-Diaminophenylether der Formel I sind literaturbekannt oder nach bekannten Syntheseverfahren zugänglich. Eine bevorzugte Verbindung, insbesondere wegen ihrer leichten Zugänglichkeit, stellt ein 2,4-Diaminophenylether oder Formel (I) dar, in der R¹, R² und R³ eine Methylgruppe sind. Ein 2,4-Diamino-3,6-dimethylanisol (R⁴ und R⁵ = H) ist z.B.- aus dem entsprechenden 2,4-Dinitro-3,6-dimethylanisol durch katalytische Hydrierung erhältlich.

Die 2,4-Diaminophenylether der Formel I kuppeln mit Entwicklerverbindungen vom Typ der p-Phenylendiamin-Derivate zu intensiven, licht- und waschechten Nuancen im Rotund Braunbereich, die auf Keratinfasern leicht und gleichgemäß aufziehen. Sie eignen sich daher ganz besonders zur Herstellung von Oxidationsfärbemitteln.

Die erfindungsgemäßen Oxidationsfärbemittel können als Entwicklerkomponente alle üblichen, bekannten Entwicklerverbindungen, z. B. die vorher beispielhaft genannten Verbindungen und Kombinationen mehrerer Entwicklerverbindungen enthalten. Zur Erzeugung natürlicher Haarfärbenuancen können neben den 2,4-Diaminophenylethern der Formel I auch weitere Kuppler in Kombination eingesetzt werden.

Die Entwickler- und Kupplerverbindungen können dabei als freie Basen oder auch in Form ihrer wasserlöslichen Säureadditionssalze, z. B. als Hydrochloride, Sulfate, Phosphate, Lactate, Acetate oder Glycolate enthalten sein.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 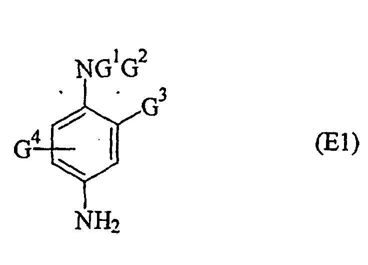 wobei
- G¹ steht für ein Wasserstoffatom, einen C₁-C₄-Alkylrest, einen C₁-C₄-Monohydroxyalkylrest, einen C₂-C₄-Polyhydroxyalkylrest, einen (C₁-C₄)-Alkoxy-(C₁-C₄)-alkylrest, einen 4'-Aminophenylrest oder einen C₁-C₄-Alkylrest, der mit einer stickstoffhaltigen Gruppe, einem Phenyl- oder einem 4'-Aminophenylrest substituiert ist,
- G² steht für ein Wasserstoffatom, einen C₁-C₄-Alkylrest, einen C₁-C₄-Monohydroxyalkylrest, einen C₂-C₄-Polyhydroxyalkylrest, einen (C₁-C₄)-Alkoxy-(C₁-C₄)-alkylrest oder einen C₁-C₄-Alkylrest, der mit einer stickstoffhaltigen Gruppe substituiert ist,
- G³ steht für ein Wasserstoffatom, ein Halogenatom, wie ein Chlor-, Brom-, Iod- oder Fluoratom, einen C₁-C₄-Alkylrest, einen C₁-C₄-Monohydroxyalkylrest, einen C₂-C₄-Polyhydroxyalkylrest, einen C₁-C₄-Hydroxyalkoxyrest, einen C₁-C₄-Acetylaminoalkoxyrest, einen C₁-C₄-Mesylaminoalkoxyrest oder einen C₁-C₄-Carbamoylaminoalkoxyrest,
- G⁴ steht für ein Wasserstoffatom, ein Halogenatom oder einen C₁-C₄-Alkylrest oder
- wenn G³ und G⁴ in ortho-Stellung zueinander stehen, können sie gemeinsam eine verbrückende α,ω-Alkylendioxogruppe, wie beispielsweise einen Ethylendioxygruppe bilden.

Beispiele für stickstoffhaltige Gruppen der Formel (E1) sind insbesondere die Aminogruppen, C₁-C₄-Monoalkylaminogruppen, C₁-C₄-Dialkylaminogruppen, C₁-C₄-Trialkylammoniumgruppen, C₁-C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-pphenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-Hydroxyethyl)amino-2-chloranilin, 2-(β-Hydroxyethyl)-pphenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenylp-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 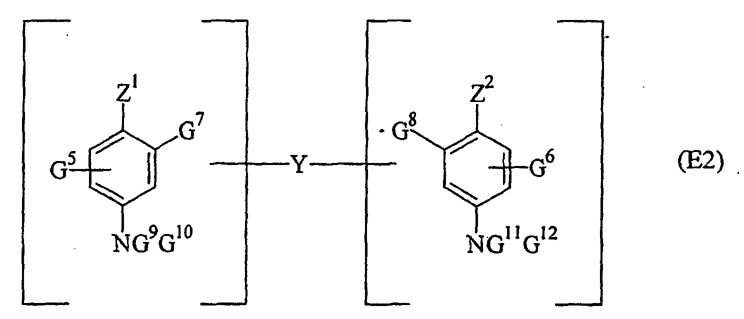 wobei:
- Z¹ und Z² stehen unabhängig voneinander für einen Hydroxyl- oder NH₂-Rest, der gegebenenfalls durch einen C₁-C₄-Alkylrest, durch einen C₁-C₄-Monohydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁-C₈-Alkoxyreste substituiert sein kann, oder eine direkte Bindung,
- G⁵ und G⁶ stehen unabhängig voneinander für ein Wasserstoff- oder Halogenatom, einen C₁-C₄-Alkylrest, einen C₁-C₄-Monohydroxyalkylrest, einen C₂-C₄-Polyhydroxyalkylrest, einen C₁-C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁-C₄-Alkylrest,
mit den Maßgaben, daß
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikemige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetra-methylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Bis-(ethyl)-N,N'-bis-(4'-amino-3'-methylphenyl)-ethylendiamin, Bis-(2-hydroxy-5-aminophenyl)-methan, 1,4-Bis-(4'-aminophenyl)-diazacycloheptan, N,N'-Bis-(2-hydroxy-5-aminobenzyl)-piperazin, N-(4'-Aminophenyl)-pphenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 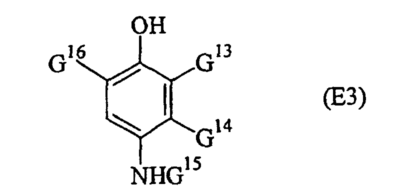 wobei
- G¹³ steht für ein Wasserstoffatom, ein Halogenatom, einen C₁-C₄-Alkylrest, einen C₁-C₄-Monohydroxyalkylrest, einen C₂-C₄-Polyhydroxyalkylrest, einen (C₁-C₄)-Alkoxy-(C₁-C₄)-alkylrest, einen C₁-C₄-Aminoalkylrest, einen Hydroxy-(C₁-C₄)-alkylaminorest, einen C₁-C₄-Hydroxyalkoxyrest, einen C₁-C₄-Hydroxyalkyl-(C₁-bis C₄)-aminoalkylrest oder einen (Di-C₁-C₄-Alkylamino)-(C₁-C₄)-alkylrest, und
- G¹⁴ steht für ein Wasserstoff- oder Halogenatom; einen C₁-C₄-Alkylrest, einen C₁-C₄-Hydroxyalkylrest, einen C₂-C₄-Polyhydroxyalkylrest, einen (C₁- C₄)-Alkoxy-(C₁-C₄)-alkylrest, einen C₁-C₄-Aminoalkylrest oder einen C₁- C₄-Cyanoalkylrest,
- G¹⁵ steht für Wasserstoff, einen C₁-C₄-Alkylrest, einen C₁-C₄-Hydroxyalkylrest, einen C₂-C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-Aminophenol, 4-Amino-3-methylphenol, 4-Amino-3-fluorphenol, 2-Hydroxymethylamino-4-aminophenol, 4-Amino-3-hydroxymethylphenol, 4-Amino-2-(2-hydroxyethoxy)phenol, 4-Amino-2-methylphenol, 4-Amino-2-hydroxymethylphenol, 4-Amino-2-methoxymethylphenol, 4-Amino-2-aminomethylphenol, 4-Amino-2-(β-hydroxyethyl-aminomethyl)phenol, 4-Amino-2-fluorphenol, 4-Amino-2-chlorphenol, 2,6-Dichlor-4-aminophenol, 4-Amino-2-((diethylamino)methyl)phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol und 4-Amino-2-((diethylamino)methyl)phenol.

Ferner kann die Entwicklerkomponente ausgewählt sein aus o-Aminophenol und seinen Derivaten, wie beispielsweise 2-Amino-4-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen. Bevorzugt werden erfindungsgemäß Pyrimidin oder Pyrazolderivate.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO - 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'chlorobenzyl)-pyrazol, 4,5-Diamino-1,3-dimethylpyrazol, 4,5-Diamino-3-methyl-1-phenylpyrazol; 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2'aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4(β-hydroxyethyl)amino-1-methylpyrazol.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 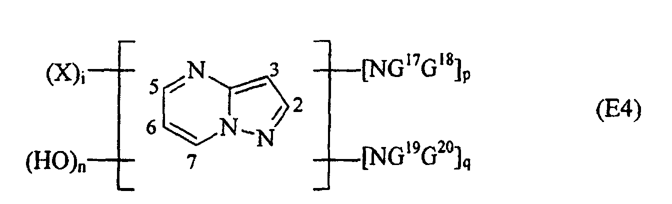 wobei:
- G¹⁷, G¹⁸, G¹⁹ und G²⁰ unabhängig voneinander stehen für ein Wasserstoffatom, einen C₁-C₄-Alkylrest, einen Aryl-Rest, einen C₁-C₄-Hydroxyalkylrest, einen C₂-C₄-Polyhydroxyalkylrest einen (C₁-C₄)-Alkoxy-(C₁-C₄)-alkylrest, einen C₁-C₄-Aminoalkylrest, der gegebenenfalls durch einen Acetyl-Ureid- oder Sulfonyl-Rest geschützt sein kann, einen (C₁-C₄)-Alkylamino-(C₁-C₄)-alkylrest, einen Di-[(C₁-C₄)-alkyl]-(C₁-C₄)-aminoalkylrest, wobei die Dialkyl-Reste gegebenenfalls einen Kohlenstoffzyklus oder einen Heterozyklus mit 5 oder 6 Kettengliedern bilden, einen C₁-C₄-Hydroxyalkyl- oder einen Di-(C₁-C₄)-[Hydroxyalkyl]-(C₁-C₄)-aminoalkylrest,
- die X-Reste stehen unabhängig voneinander für ein Wasserstoffatom, einen C₁-C₄-Alkylrest, einen Aryl-Rest, einen C₁-C₄-Hydroxyalkylrest, einen C₂-C₄-Polyhydroxyalkylrest, einen C₁-C₄-Aminoalkylrest, einen (C₁-C₄)-Alkylamino-(C₁-C₄)-alkylrest, einen Di-[(C₁-C₄)alkyl]- (C₁-C₄)-aminoalkylrest, wobei die Dialkyl-Reste gegebenenfalls einen Kohlenstoffzyklus oder einen Heterozyklus mit 5 oder 6 Kettengliedern bilden, einen C₁-C₄-Hydroxyalkyl- oder einen Di-(C₁-C₄hydroxyalkyl)-aminoalkylrest, einen Aminorest, einen C₁-C₄-Alkyl- oder Di-(C₁-C₄hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, daß
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

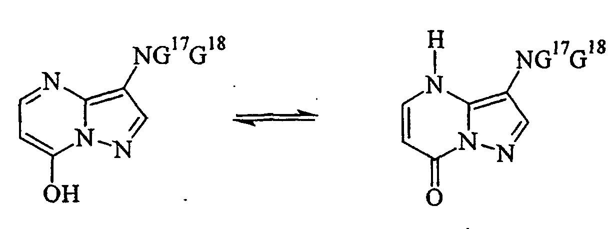

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl pyrazol-[1,5-a]-pyrimidin-3,7-diamin;
- Pyrazol-(1,5-a]-pyrimidin-3,5-diamin;
- 2,7-Dimethyl pyrazol-[1,5-a]-pyrimidin-3,5-diamin;
- 3-Amino pyrazol-[1,5-a]-pyrimidin-7-ol;
- 3-Amino pyrazol-[1,5-a]-pyrimidin-5-ol;
- 2-(3-Amino pyrazol-[1,5-a]-pyrimidin-7-ylamino)-ethanol;
- 2-(7-Amino pyrazol-[1,5-a]-pyrimidin-3-ylamino)-ethanol;
- 2-[(3-Amino pyrazol-[1,5-a]-pyrimidin-7-yl)-(2-hydroxy-ethyl)-amino]-ethanol;
- 2-[(7-Amino pyrazol-[1,5-a]-pyrimidin-3-yl)-(2-hydroxy-ethyl)-amino]-ethanol;
- 5,6-Dimethyl pyrazol-[1,5-a]-pyrimidin-3,7-diamin;
- 2,6-Dimethyl pyrazol-[1,5-a]-pyrimidin-3,7-diamin;
- 2,5, N7, N7-Tetramethyl pyrazol-[1,5-a]-pyrimidin-3,7-diamin;
sowie ihre physiologisch verträglichen Salze und ihre tautomeren Formen, wenn ein tautomeres Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

Wegen der besonders interessanten Rot- und Brauntöne sind als Entwicklerkomponenten besonders bevorzugt p-Phenylendiamin oder p-Phenylendiaminderivate oder Salze davon enthalten. Dabei handelt es sich entweder um einkernige, bevorzugt substituierte p-Phenylendiamine oder um mehrkernige Verbindungen, bei denen zwei gegebenenfalls substituierte Paraphenylendiamin-Keme über eine Alkylen-, Hydroxyalkylen- oder Alkylendioxybrücke zwischen den Kernen in Position 2 oder über Alkylen- oder Hydroxyalkylenbrücken zwischen den Stickstoffatomen in Position 1 miteinander verknüpft sind.
Beispiele für solche geeigneten p-Phenylendiamine sind z. B. p-Phenylendiamin, p-Toluylendiamin, 2,5-Dimethyl-p-phenylendiamin, 2,6-Dimethyl-p-phenylendiamin, 2-(2',5'-Diaminophenyl)-ethanol, 2-(2',5'-Diaminophenoxy)-ethanol, N,N-Bis-(2'hydroxyethyl)-p-phenylendiamin, N,N'-Bis-(2'-hydroxyethyl)-p-phenylendiamin, 4-N,N-Dimethylaminoanilin, N,N'-Bis-(2'-hydroxyethyl)- N,N'-(4'-aminophenyl)-1,3-diaminopropan-2-ol, 1,3-Bis-(2',5'-diaminophenyl)-propan, 1,3-Bis-(2',5'diaminophenoxy)-propan, Bis-(2,5-diaminophenyl)-methan und 4,4'-Diamino-diphenylamin.

In einer speziellen Ausführungsform sind zur weiteren Nuancierung weitere Entwicklerverbindungen geeignet, wie p-Aminophenol, o-Aminophenol, 4-Amino-3-methylphenol, 4-Amino-2-methylphenol, 2-Hydroxymethyl-4-aminophenol, 2-Hydroxyethylaminomethyl-4-aminophenol, 4-Amino-3-fluorphenol, 2-Hydroxyethoxy-4-aminophenol, 1-(2'-Hydroxy-5'-aminobenzyl)-imidazolin-2-on, 1-Phenyl-3-carboxyamido-4-aminopyrazolin-5, 4,5-Diamino-1-(2'-hydroxyethyl)-pyrazol, N,N'-Bis-(2'-hydroxy-5'-aminobenzyl)-piperazin, 2,4,5,6-Tetraaminopyrimidin, 2-Dimethylamino-4,5,6-tetraaminopyrimidin, 2-Hydroxy-4,5,6-triaminopyrimidin, 4-Hydroxy-2,5,6-triaminopyrimidin oder 2,4-Dihydroxy-5,6-diaminopyrimidin.

Als weitere Kupplersubstanzen werden beispielsweise gegebenenfalls m-Phenylendiaminderivate, Naphthole, Resorcin und Resorcinderivate, Pyrazolone und m-Aminophenole verwendet.

Erfindungsgemäß bevorzugte weitere Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoracetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2.4-Diaminophenoxyethanol, 1,3-Bis-(2',4'-diaminophenoxy)propan, 1-Methoxy-2-amino-4-(2'-hydroxyethylamino)-benzol, 1,3-Bis-(2',4'-diaminophenyl)propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

In einer speziellen Ausführungsform sind geeignete weitere Kupplerkomponenten ausgewählt aus: 2,7- und 1,7-Dihydroxynaphthalin, o-Aminophenol, 5-Amino-2-methylphenol, m-Aminophenol, Resorcin, Resorcinmonomethylether, m-Phenylendiamin, 5-Chlor-2,4-diaminotoluol, 1-Phenyl-3-methyl-pyrazolon-5, 2,4-Dichlor-3-aminophenol, 1,3-Bis-(2',4'-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 2,6-Bis-(2'hydroxyethylamino)-toluol, 2,4-Diaminophenoxyethanol, 1-Methoxy-2-amino-4-(2'hydroxyethylamino)-benzol, 2-Methyl-4-chlor-5-amino-phenol, 6-Methyl-1,2,3,4-tetrahydro-chinoxalin, 3,4-Methylendioxyphenol, 3,4-Methylendioxyanilin, 2,6-Dimethyl-3-amino-phenol, 3-Amino-6-methoxy-2-methylaminophenol, 2-Hydroxy-4-aminophenoxyethanol, 2-Methyl-5-(2'-hydroxyethylamino)-phenol und 2,6-Dihydroxy-3,4-dimethylpyridin.

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel.

Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einemmol-Verhältnis von 1:0,5 bis 1:3, insbesondere 1:1 bis 1:2, enthalten sein können.

Eine natürlich erscheinende Haarfarbe kann aus einem Färbevorgang hervorgehen, wenn in dem applizierten Haarfärbemittel zusätzlich Indol- oder Indolinderivate als Vorprodukte naturanaloger Farbstoffe verwendet werden.

In einer weiteren Ausführungsform werden daher zusätzlich bevorzugt Indole und/oder Indoline eingesetzt, die mindestens eine Hydroxy- oder Aminogruppe, bevorzugt als Substituent am. Sechsring, aufweisen. Diese Gruppen können weitere Substituenten tragen, z. B. in Form einer Veretherung oder Veresterung der Hydroxygruppe oder eine Alkylierung der Aminogruppe.

Besonders gut als Vorstufen naturanaloger Haarfarbstoffe geeignet sind Derivate des 5,6-Dihydroxyindolins der Formel (IIa), 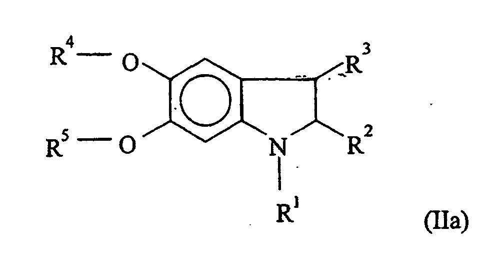 in der unabhängig voneinander
R¹ steht für Wasserstoff, eine C₁-C₄-Alkylgruppe, eine C₃-C₆-Cycloalkylgruppe, eine C₂-C₄-Alkenylgruppe oder eine C₁-C₄-Hydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der R⁶ steht für eine C₁-C₄-Alkylgruppe, und.
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IIb), 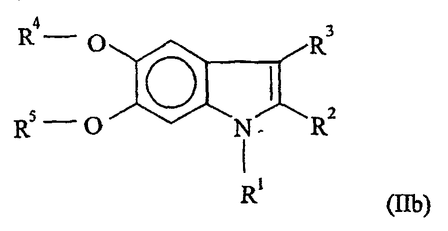 in der unabhängig voneinander
R¹ steht für Wasserstoff, eine C₁-C₄-Alkylgruppe, eine C₃-C₆-Cycloalkylgruppe, eine C₂-C₄-Alkenylgruppe oder eine C₁-C₄-Hydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

Bevorzugt enthalten die erfindungsgemäßen Haarfärbemittel zur weiteren Modifizierung der Farbnuancen zusätzlich folgende übliche direktziehende Farbstoffe: Nitrophenylendiamine, Nitroaminophenole, Azofarbstoffe, Anthrachinone oder Indophenole. Bevorzugte direktziehende Farbstoffe sind unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 12, Disperse Blue 3, HC Violet 1, Disperse Violet 1, Disperse Violet 4, Acid Violet 43, Disperse Black 9 und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol bekannt.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

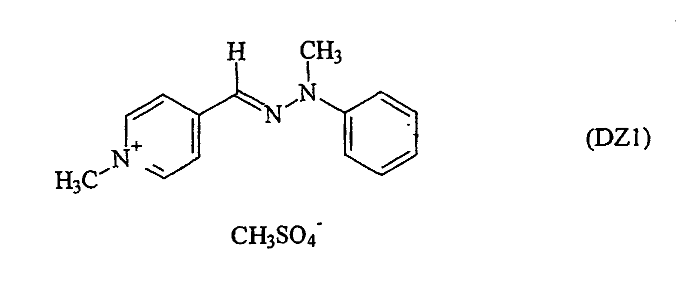

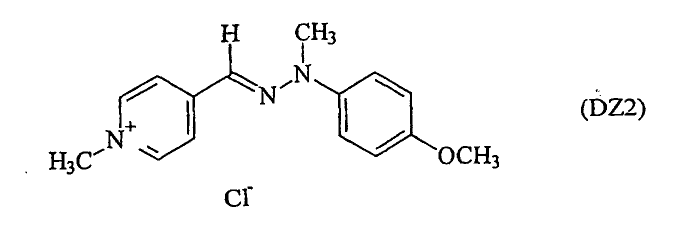

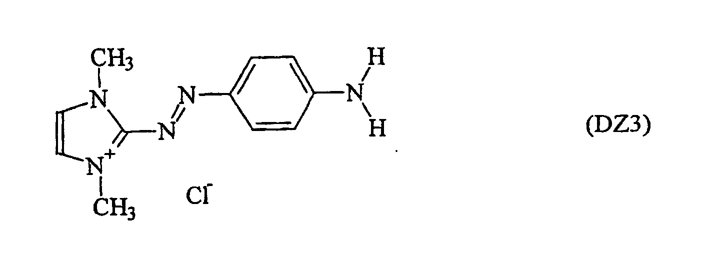

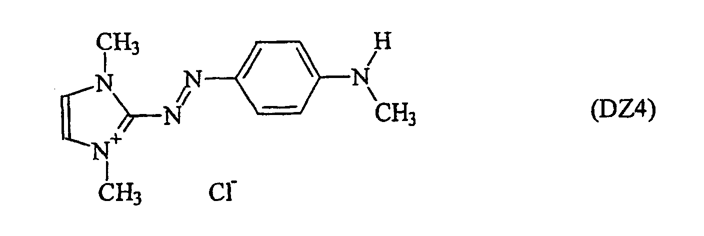

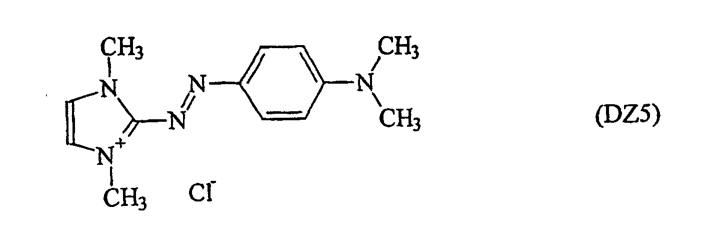

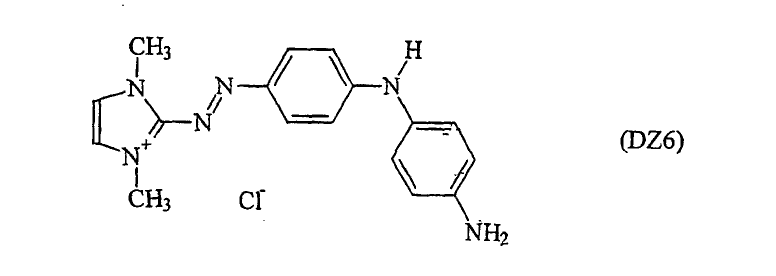

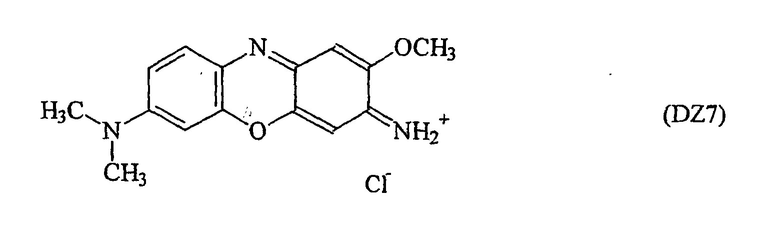

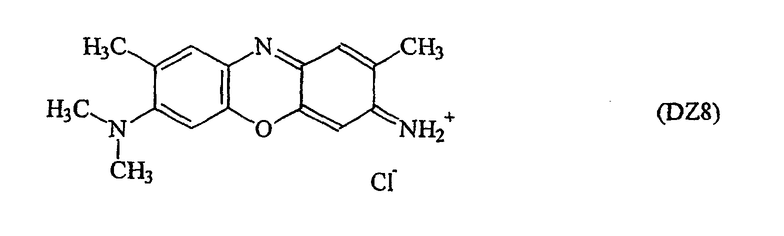

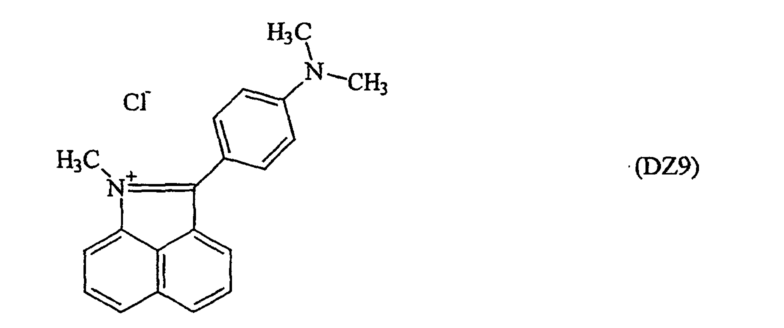

Die Verbindungen der Formeln (DZ1), (DZ3) und (DZ5) sind ganz besonders bevorzugte kationische direktziehende Farbstoffe der Gruppe (c). Die kationischen direktziehenden Farbstoffe, die unter dem Warenzeichen Arianor® vertrieben werden, sind erfindungsgemäß besonders bevorzugte direktziehende Farbstoffe.

In einer speziellen Ausführungsform enthalten die erfindungsgemäßen Haarfärbemittel zur weiteren Modifizierung der Farbnuancen neben den Oxidationsfarbstoffvorprodukten zusätzlich folgende übliche direktziehende Farbstoffe: Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol.

Die erfindungsgemäßen Mittel gemäß dieser- Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe), sowie Kapitel 8, Seiten 264-267; Oxidationsfarbstoffvorprodukte), erschienen als Band 7 der Reihe "Dermatology" (Hrsg.: Ch. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Zur Herstellung der erfindungsgemäßen Oxidationsfärbemittel werden die Oxidationsfarbstoffvorprodukte in einen geeigneten wäßrigen Träger eingearbeitet. Solche Träger sind z.B. verdickte wäßrige Lösungen, Cremes (Emulsionen), Gele oder tensidhaltige schäumende Zubereitungen, z.B. Shampoos oder Schaumaerosole oder andere Zubereitungen, die für die Anwendung auf dem Haar geeignet sind. Als Träger eignen sich auch wasserfreie Pulver, in diesem Falle wird die Zubereitung vor der Anwendung auf der Faser in Wasser gelöst oder dispergiert.

Als Trägerkomponenten werden bevorzugt
- Netz- und Emulgiermittel
- Verdickungsmittel
- Reduktionsmittel (Antioxidantien)
- haarpflegende Zusätze
- Duftstoffe und
- Lösungsmittel wie z. B. Wasser, Glycole oder niedere Alkohole
eingesetzt.

Als Netz- und Emulgiermittel eignen sich z. B. anionische, zwitterionische, ampholytische und nichtionische Tenside. Auch kationische Tenside können zur Erzielung bestimmter Effekte eingesetzt werden.
In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z.B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß-DE A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R'O-(Z)ₓ. Diese Verbindungen sind durch die folgenden Parameter gekennzeichnet.

Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1 -Myristyl, 1-Cetyl und 1-Stearyl. Besonders bevorzugt sind 1-Octyl, 1-Decyl, 1-Lauryl, 1-Myristyl.

Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R' enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈-C₁₆-Alkylgruppen oder
- im wesentlichen aus C₁₂-C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine
-COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethyl-ammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Als Verdickungsmittel eignen sich einmal die wasserlöslichen hochmolekularen Polysaccharid-Derivate oder Polypeptide, z. B. Cellulose- oder Stärkeether, Gelatine, Pflanzengumme, Biopolymere (Xanthan-Gum) oder wasserlösliche synthetische Polymere wie z. B. Polyvinylpyrrolidon, Polyvinylalkohol, Polyethylenoxide, Polyacrylamide, Polyurethane, Polyacrylate und andere. Zum anderen kann man tensidhaltige Zubereitungen auch durch Solubilisierung oder Emulgierung von polaren Lipiden verdicken. Solche Lipide sind z. B. Fettalkohole mit 12 - 18 C-Atomen, (freie) Fettsäuren mit 12 - 18 C-Atomen, Fettsäurepartialglyceride, Sorbitanfettsäureester. Fettsäurealkanolamide, niedrig oxethylierte Fettsäuren oder Fettalkohole, Lecithine, Sterine. Schließlich kann man gelförmige Träger auch auf Basis wässriger Seifengele, z. B. von Ammonium-Oleat, erzeugen. Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol werden bevorzugt verwendet,

Reduktionsmittel (Antioxidantien), die dem Träger zugesetzt werden, um eine vorzeitige oxidative Entwicklung des Farbstoffs vor der Anwendung auf dem Haar zu verhindern, sind z. B. Natriumsulfit oder Natriumascorbat.

Haarpflegende Zusätze können z. B. Fette, Öle oder Wachse in emulgierter Form, strukturgebende Additive wie z. B. Glucose oder Pyridoxin, avivierende Komponenten wie z. B. wasserlösliche Proteine, Proteinabbauprodukte, Aminosäuren, wasserlösliche kationische Polymere, Silicone, Vitamine, Panthenol oder Pflanzenextrakte sein.

Schließlich können Duftstoffe und Lösungsmittel wie z. B. Glycole wie 1,2-Propylenglycole, Glycerin, Glycolether wie z. B. Butylglycol, Ethyldiglycol oder niedere einwertige Alkohole wie Ethanol oder Isopropanol enthalten sein.

Zusätzlich können noch weitere Hilfsmittel enthalten sein, die die Stabilität und Anwendungseigenschaften der Oxidationsfärbemittel verbessern, z. B. Komplexbildner wie EDTA, NTA oder Organophosphonate, Quell- und Penetrationsmittel wie z. B. Harnstoff, Guanidin, Hydrogencarbonate, Puffersalze wie z. B. Ammoniumchlorid, Ammoniumcitrat, Ammoniumsulfat oder Alkanolammoniumsalze und gegebenenfalls Treibgase.

Weiterhin können die erfindungsgemäßen Mittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat®100 (Poly(dimethyldiallylammoniumchlorid)), Merquat®550 (Dimethyldiallylammoniumchlorid-Acrylamid-Copolymer) und Merquat® 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat®734 und Gafquat®755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquatemium-2, Polyquaternium-10 und Polyquaternium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Coming unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning® 929-Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate, Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem; Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie den eingesetzten Mengen dieser Komponenten wird ausdrücklich auf die dem Fachmann bekannten einschlägigen Handbücher, z. B. K. Schrader, Grundlagen und Rezepturen der Kosmetika, 2. Auflage, Hüthig Buch Verlag, Heidelberg, 1989, verwiesen.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Man kann aber das Oxidationsfärbemittel auch zusammen mit einem Katalysator auf das Haar aufbringen, der die Oxidation der Farbstoffvorprodukte, z.B. durch Luftsauerstoff, aktiviert. Solche Katalysatoren sind z.B. Übergangsmetallverbindungen, Iodide, Chinone oder bestimmte Enzyme. Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Ein weiterer Gegenstand der Erfindung ist daher ein Verfahren zum Färben von Keratinfasern, insbesondere von Haaren, bei dem man auf die Faser ein erfindungsgemäßes Oxidationsfärbemittel zusammen mit einem Oxidationsmittel und/oder mit einem Katalysator zur Aktivierung der Oxidation aufbringt und nach einer Einwirkungszeit wieder mit Wasser oder mit einer wäßrigen Tensidzubereitung abspült. Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Die nachfolgenden Beispiele sollen den Erfindungsgegenstand näher erläutern.

### Beispiele

Es wurde eine Basis-Creme der folgenden Zusammensetzung hergestellt:

| | |
|---|---|
| Hydrenol®D¹ | 17,00 g |
| Lorol®techn.² | 4,00 g |
| Eumulgin®B2³ | 1,50 g |
| Texapon®NSO⁴ | 40,00 g |
| Dehyton®K⁵ | 25,00 g |
| Wasser | 12,50 g |

| | |
|---|---|
| ¹ C₁₆-C₁₈-Fettalkohol (INCI-Bezeichnung: Cetearyl alcohol) (COGNIS) | |
| ² C₁₂-C₁₈-Fettalkohol (INCI-Bezeichnung: Coconuf alcohol) (COGNIS) | |
| ³ Cetearylstearylalkohol mit ca. 20 EO-Einheiten (INCI-Bezeichnung: Ceteareth-20) (COGNIS) | |
| ⁴ Laurylethersulfat, Natriumsalz (ca. 27,5 % Aktivsubstanz; (INCI-Bezeichnung: Sodium Laureth Sulfate) (COGNIS) | |
| ⁵ N,N-Dimethyl-N-(C₈-C₁₈-kokosamidopropyl)ammoniumacetobetain (ca. 30 % Aktivsubstanz; INCI-Bezeichnung: Aqua (Water), Cocamidopropyl Betaine) (COGNIS) | |

Die Substanzen Hydrenol®D, Lorol® und Eumulgin®B2 wurden bei 80°C aufgeschmolzen, mit dem 80°C heißem Wasser, enthaltend Texapon®NSO und Dehyton®K, vermischt und unter starkem Rühren emulgiert. Danach wurde die Emulsion unter schwachem Rühren abgekühlt.

Es wurden Färbecremes der folgenden Zusammensetzung hergestellt:

| | |
|---|---|
| Basiscreme | 50,0 g |
| Entwicklerkomponente | 7,5 mmol |
| 2,4-Diamino-3,6-dimethylanisol (erfindungsgemäß) | 1,173 g (7,5 mmol) |
| Na₂SO₃ (Inhibitor) | 1,0 g |
| (NH₄)₂SO₄ | 1,0 g |
| Konz. NH₃-Lösung | ad pH=10 |
| Wasser | ad 100 g |

Für die Ausfärbung wurden folgende Entwicklerverbindungen verwendet:
E1: p-Toluylendiamin
E2: 2,5-Diaminophenylethanol
E3: N,N'-Bis-(2'-hydroxyethyl)-p-phenylendiamin
E4: 1,3-N,N'-Bis-(2'-hydroxyethyl)-N,N'-bis-(4'-aminophenyl)-diaminopropan-2-ol.
E5: p-Aminophenol
E6: 2,4,5,6-Tetraaminopyrimidin.

Die Bestandteile werden der Reihe nach miteinander vermischt. Nach Zugabe der Oxidationsfarbstoffvorprodukte, des Inhibitors und des (NH₄)₂SO₄ wurde mit konzentrierter Ammoniaklösung der pH-Wert der Emulsion auf 10 eingestellt, dann wurde mit Wasser auf 100g aufgefüllt.

Die Entwicklung der Färbung wurde mit 1 Gew.-%iger Wasserstoffperoxidlösung durchgeführt. Hierzu wurden 100g der Emulsion mit 50g H₂O₂-Lösung (1%ig) vermischt.

Die gebrauchsfertigen Färbeansätze wurden dann auf 5 cm lange Strähnen standardisierten, zu 80 % ergrauten, aber nicht besonders vorbehandelten Menschenhaars (Fa. Kerling) aufgetragen.

Nach 30 Minuten Einwirkzeit bei 32°C wurde das.Haar mit Wasser gespült, mit einem üblichen Shampoo ausgewaschen, erneut gespült und getrocknet.

Die Ergebnisse der Färbeversuche sind der Tabelle zu entnehmen:

| **Beispiel** | **Entwickler** | **enthaltene Nuance** |
|---|---|---|
| 1 | E1 | englischrot |
| 2 | E2 | terra di Siena |
| 3 | E3 | kardinalrot |
| 4 | E4 | violettbraun |
| 5 | E5 | braun |
| 6 | E6 | bernsteingelb |

## Patentansprüche

1. Oxidationsfärbemittel zum Färben von Keratinfasern, insbesondere von Haaren, die in einem wässrigen Träger wenigstens eine Entwicklerkomponente und als Kupplerkomponente wenigstens einen 2,4-Diaminophenylether der Formel (I), 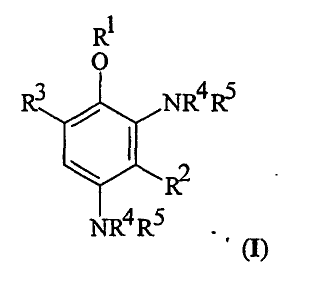 in der
• R¹ steht für eine C₁-C₄-Alkylgruppe, eine C₁-C₄-Monohydroxyalkylgruppe, eine C₂-C₄-Polyhydroxyalkylgruppe oder eine Phenylgruppe,
• R² und R³ unabhängig voneinander stehen für eine C₁-C₄-Alkylgruppe, eine C₁-C₄-Monohydroxyalkylgruppe oder eine C₂-C₄-Polyhydroxyalkylgruppe und
• R⁴ und R⁵ unabhängig voneinander stehen für Wasserstoff, eine C₁-C₄-Alkylgruppe, eine C₂-C₄-Monohydroxyalkylgruppe, eine C₂-C₄-Polyhydroxyalkylgruppe, eine Allylgruppe oder eine Benzylgruppe,
oder deren Salze enthalten.

2. Oxidationsfärbemittel gemäß Anspruch 1, **dadurch gekennzeichnet, daß** sie als Kupplerkomponente wenigstens einen 2,4-Diaminophenylether der Formel (I), in der R¹ steht für eine C₁-C₄-Alkyl- oder eine C₂-C₄-Monohydroxyalkylgruppe, enthalten.

3. Oxidationsfärbemittel gemäß einem der Ansprüche 1 oder 2, **dadurch gekennzeichnet, daß** sie als Kupplerkomponente wenigstens einen 2,4-Diaminophenylether der Formel (I), in der R² und R³ stehen unabhängig voneinander für eine C₁-C₄-Alkyl- oder Monohydroxyalkylgruppe, enthalten.

4. Oxidationsfärbemittel gemäß einem der Ansprüche 1 bis 3, **dadurch gekennzeichnet, daß** sie als Kupplerkomponente wenigstens einen 2,4-Diaminophenylether der Formel (I), in der R⁴ und R⁵ stehen für Wasserstoff, enthalten.

5. Oxidationsfärbemittel gemäß einem der Ansprüche 1 bis 4, **dadurch gekennzeichnet, daß** als Kupplerkomponente wenigstens ein 2,4-Diaminophenylether der Formel (I), in der R¹, R² und R³ eine Methylgruppe sind sowie R⁴ und R⁵ für Wasserstoff stehen, oder ein Salz davon enthalten ist.

6. Oxidationsfärbemittel gemäß einem der Ansprüche 1 bis 5, **dadurch gekennzeichnet, daß** als Entwicklerkomponente p-Phenylendiamin oder ein p-Phenylendiaminderivat oder ein Salz davon enthalten ist.

7. Oxidationsfärbemittel gemäß einem der Ansprüche 1 bis 6, **dadurch gekennzeichnet, daß** die Entwicklerkomponente ausgewählt ist aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-pphenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-Hydroxyethyl)amino-2-chloranilin, 2-(β-Hydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-pphenylendiamin, N-(4'-Aminophenyl)-p-phenylendiamin, N-Phenyl-pphenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, 5,8-Diaminobenzo-1,4-dioxan, N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetra-methylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Bis-(ethyl)-N,N'-bis-(4'-amino-3'methylphenyl)-ethylendiamin, 1,4-Bis-(4'-aminophenyl)-diaza-cycloheptan, N-(4'-Aminophenyl)-p-phenylendiamin und 1,10-Bis-(2',5'-diaminophenyl)-1,4,7,10-tetraoxadecan sowie ihren physiologisch verträglichen Salzen.

8. Oxidationsfärbemittel gemäß einem der Ansprüche 1 bis 7, **dadurch gekennzeichnet, daß** die Entwicklerkomponente ausgewählt ist aus p-Phenylendiamin, p-Toluylendiamin, 2,5-Dimethyl-p-phenylendiamin, 2,6-Dimethyl-p-phenylendiamin, 2-(2',5'-Diaminophenyl)-ethanol, 2-(2',5'-Diaminophenoxy)-ethanol, N,N-Bis-(2'-hydroxyethyl)-p-phenylendiamin, N,N'-Bis-(2'-hydroxyethyl)-p-phenylendiamin, 4-N,N-Dimethylaminoanilin, N,N'-Bis-(2'hydroxyethyl)-N,N'-(4'-aminophenyl)-1,3-diaminopropan-2-ol, 1,3-Bis-(2',5'-diaminophenyl)-propan, 1,3-Bis-(2',5'-diaminophenoxy)-propan, Bis-(2,5-diaminophenyl)-methan und 4,4'-Diamino-diphenylamin.

9. Oxidationsfärbemittel gemäß einen der Ansprüche 1 bis 8, **dadurch gekennzeichnet, daß** das die Entwicklerkomponenten als auch die Kupplerkomponenten bevorzugt in einer Menge von 0,005 bis 20 Gew.-% jeweils bezogen auf das gesamte Oxidationsfärbemittel enthalten sind.

10. Oxidationsfübemittel gemäß einen der Ansprüche 1 bis 9, **dadurch gekennzeichnet, daß** mindestens ein Indol- und/oder Indolinderivat enthalten ist.

11. Oxidationsfärbemittel gemäß einen der Ansprüche 1 bis 10, **dadurch gekennzeichnet, daß** der kosmetische Träger eine Creme, eine Emulsion, ein Gel, ein Shampoo oder ein Schaumaerosol ist.

12. Verfahren zur Färbung von Keratinfasern, insbesondere von Haaren, **dadurch gekennzeichnet, daß** man auf die Fasern ein Oxidationsfärbemittel gemäß einem der Ansprüche 1 bis 11 zusammen mit einem Oxidationsmittel und/oder mit einem Katalysator zur Aktivierung der Oxidation aufbringt und nach einer Einwirkungszeit wieder mit Wasser oder einer wässrigen Tensidzubereitung abspült.

## Claims

1. Oxidation colorants for colouring keratin fibres, more particularly hair, which contain in a water-based carrier at least one primary intermediate and, as secondary intermediate, at least one 2,4-diaminophenylether corresponding to formula (I): 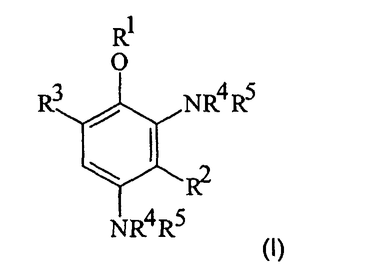 in which
• R¹ is a C₁₋₄ alkyl group, a C₁₋₄ monohydroxyalkyl group, a C₂₋₄ polyhydroxyalkyl group or a phenyl group,
• R² and R³ independently of one another represent a C₁₋₄ alkyl group, a C₁₋₄ monohydroxyalkyl group or a C₂₋₄ polyhydroxyalkyl group and
• R⁴ and R⁵ independently of one another represent hydrogen, a C₁₋₄ alkyl group, a C₂₋₄ monohydroxyalkyl group, a C₂₋₄ polyhydroxyalkyl group, an allyl group or a benzyl group,
or salts thereof.

2. Oxidation colorants as claimed in claim 1, **characterized in that** they contain at least one 2,4-diaminophenylether corresponding to formula (I), in which R¹ is a C₁₋₄ alkyl group or a C₂₋₄ monohydroxyalkyl group, as secondary intermediate.

3. Oxidation colorants as claimed in claim 1 or 2, **characterized in that** they contain at least one 2,4-diaminophenylether corresponding to formula (I), in which R² and R³ independently of one another represent a C₁₋₄ alkyl or monohydroxyalkyl group, as secondary intermediate.

4. Oxidation colorants as claimed in any of claims 1 to 3, **characterized in that** they contain at least one 2,4-diaminophenylether corresponding to formula (I), in which R⁴ and R⁵ represent hydrogen, as secondary intermediate.

5. Oxidation colorants as claimed in any of claims 1 to 4, **characterized in that** at least one 2,4-diaminophenylether corresponding to formula (I), in which R¹, R² and R³ represent a methyl group and R⁴ and R⁵ represent hydrogen, or a salt thereof is present as secondary intermediate.

6. Oxidation colorants as claimed in any of claims 1 to 5, **characterized in that** p-phenylenediamine or a p-phenylenediamine derivative or a salt thereof is present as primary intermediate.

7. Oxidation colorants as claimed in any of claims 1 to 6, **characterized in that** the primary intermediate is selected from p-phenylenediamine, p-toluylenediamine, 2-chloro-p-phenylenediamine, 2,3-dimethyl-p-phenylenediamine, 2,6-dimethyl-p-phenylenediamine, 2,6-diethyl-p-phenylenediamine, 2,5-dimethyl-p-phenylenediamine, N,N-dimethyl-p-phenylenediamine, N,N-diethyl-p-phenylenediamine, N,N-dipropyl-p-phenylenediamine, 4-aamino-3-methyl-(N,N-diethyl)-aniline, N,N-bis-(β-hydroxyethyl)-p-phenylenediamine, 4-N,N-bis-(β-hydroxyethyl)-amino-2-methylaniline, 4-N,N-bis-(β-hydroxyethyl)-amino-2-chloroaniline, 2-(β-hydroxyethyl)-p-phenylenediamine, 2-fluoro-p-phenylenediamine, 2-isopropyl-p-phenylenediamine, N-(β-hydroxypropyl)-p-phenylenediamine, 2-hydroxymethyl-p-phenylenediamine, N,N-dimethyl-3-methyl-p-phenylenediamine, N,N-(ethyl-β-hydroxyethyl)-p-phenylenediamine, N-(β,γ-dihydroxypropyl)-p-phenylenediamine, N-(4'-aminophenyl)-p-phenylenediamine, N-phenyl-p-phenylenediamine, 2-(β-hydroxyethyloxy)-p-phenylenediamine, 2-(β-acetylaminoethyloxy)-p-phenylenediamine, N-(β-methoxyethyl)-p-phenylenediamine, 5,8-diaminobenzo-1,4-dioxane, N,N'-bis-(β-hydroxyethy))-N,N'-bis-(4'-aminophenyl)-1,3-diaminopropan-2-ol, N,N'-bis-(β-hydroxyethyl)-N,N'-bis-(4'-aminophenyl)-ethylenediamine, N,N'-bis-(4-aminophenyl)-tetramethylenediamine, N,N'-bis-(β-hydroxyethyl)-N,N'-bis-(4-aminophenyl)-tetramethylenediamine, N,N'-bis-(4-methyl-aminophenyl)-tetramethylenediamine, N,N'-bis-(ethyl)-N,N'-bis-(4'-amino-3'-methylphenyl)-ethylenediamine, 1,4-bis-(4'-aminophenyl)-diaza-cycloheptane, N-(4'-aminophenyl)-p-phenylenediamine and 1,10-bis-(2',5'-diaminophenyl)-1,4,7,10-tetraoxadecane and physiologically compatible salts thereof.

8. Oxidation colorants as claimed in any of claims 1 to 7, **characterized in that** the primary intermediate is selected from p-phenylenediamine, p-toluylenediamine, 2,5-dimethyl-p-phenylenediamine, 2,6-dimethyl-p-phenylenediamine, 2-(2',5'-diaminophenyl)-ethanol, 2-(2',5'-diaminophenoxy)-ethanol, N,N-bis-(2'-hydroxyethyl)-p-phenylenediamine, N,N'-bis-(2'-hydroxyethyl)-p-phenylenediamine, 4-N,N-dimethylaminoaniline, N,N'-bis-(2'-hydroxyethyl)-N,N'-(4'-aminophenyl)-1,3-diaminopropan-2-ol, 1,3-bis-(2',5'-diaminophenyl)-propane, 1,3-bis-(2',5'-diaminophenoxy)-propane, bis-(2,5-diaminophenyl)-methane and 4,4'-diaminodiphenylamine.

9. Oxidation colorants as claimed in any of claims 1 to 8, **characterized in that** both the primary intermediates and the secondary intermediates are preferably used in a quantity of 0.005 to 20% by weight, based on the oxidation colorant as a whole.

10. Oxidation colorants as claimed in any of claims 1 to 9, **characterized in that** at least one indole and/or indoline derivative is/are present.

11. Oxidation colorants as claimed in any of claims 1 to 10, **characterized in that** the cosmetic carrier is a cream, an emulsion, a gel, a shampoo or a foam aerosol.

12. A process for coloring keratin fibres, more particularly hair, **characterized in that** the oxidation colorant claimed in any of claims 1 to 11 is applied to the fibres together with an oxidizing agent and/or a catalyst for activating oxidation and, after a contact time, is rinsed off again with water or a water-based surfactant preparation.

## Revendications

1. Agents de teinture d'oxydation pour teindre des fibres kératiniques, en particulier les cheveux, qui contiennent dans un support aqueux au moins un composant de développement et comme composant de couplage au moins un 2,4-diaminophényléther de formule (I), 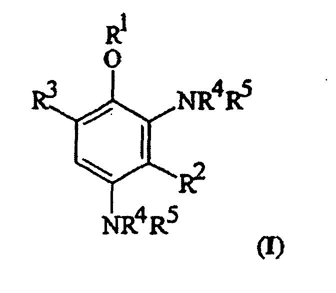 dans laquelle
• R¹ représente un groupe alkyle en C₁ à C₄ ,un groupe monohydroxyalkyle en C₁ à C₄, un groupe polyhydroxyalkyle en C₂ à C₄ ou un groupe phényle,
• R² et R³ représentent, indépendamment l'un de l'autre, un groupe alkyle en C₁ à C₄, un groupe monohydroxyalkyle en C₁ à C₄, ou un groupe polyhydroxyalkyle en C₂ à C₄ et
• R⁴ et R⁵ représentent, indépendamment l'un de l'autre, hydrogène, un groupe alkyle en C₁ à C₄, un groupe monohydroxyalkyle en C₂ à C₄, un groupe polyhydroxyalkyle en C₂ à C₄ , un groupe allyle ou un groupe benzyle
ou ses sels.

2. Agents de teinture d'oxydation selon la revendication 1, **caractérisés en ce qu'**ils contiennent comme composant de couplage au moins un 2,4-diaminophényléther de formule (I), dans laquelle R¹ représente un groupe alkyle en C₁ à C₄ ou un groupe monohydroxyalkyle en C₂ à C₄.

3. Agents de teinture d'oxydation selon l'une quelconque des revendications 1 ou 2, **caractérisés en ce qu'**ils contiennent comme composant de couplage au moins un 2,4-diaminophényléther de formule (1), dans laquelle R² et R³ représentent, indépendamment l'un de l'autre, un groupe alkyle ou monohydroxyalkyle en C₁ à C₄.

4. Agents de teinture d'oxydation selon l'une quelconque des revendications 1 à 3, **caractérisés en ce qu'**ils contiennent comme composant de couplage au moins un 2,4-diaminophényléther de formule (I), dans laquelle R⁴ et R⁵ représentent hydrogène.

5. Agents de teinture d'oxydation selon l'une quelconque des revendications 1 à 4, **caractérisés en ce qu'**ils contiennent comme composant de couplage au moins un 2,4-diaminophényléther de formule (1), dans laquelle R¹, R² et R³ représentent un groupe méthyle et R⁴ et R⁵ représentent hydrogène, ou un sel de celui-ci.

6. Agents de teinture d'oxydation selon l'une quelconque des revendications 1 à 5, **caractérisés en ce qu'**ils contiennent comme composant de développement la p-phénylènediamine ou un dérivé de la p-phénylènediamine ou un sel de celui-ci.

7. Agents de teinture d'oxydation selon l'une quelconque des revendications 1 à 6, **caractérisés en ce que** le composant de développement est choisi parmi la p-phénylènediamine, la p-toluylènediamine, la 2-chloro-p-phénylènediamine, la 2,3-diméthyl-p-phénylènediamine, la 2,6-diméthyl-p-phénylènediamine, la 2,6-diéthyl-p-phénylènediamine, la 2,5-diméthyl-p-phénylènediamine, la N,N-diméthyl-p-phénylènediamine, la N,N-diéthyl-p-phénylènediamine, la N,N-dipropyl-p-phénylènediamine, la 4-amino-3-méthyl-(N,N-diéthyl)-aniline, la N,N-bis-(β-hydroxyéthyl)-p-phénylènediamine, la 4-N,N-bis-(β-hydroxyéthyl)amino-2-méthylaniline, la 4-N,N-bis-(β-hydroxyéthyl)amino-2-chloroaniline, la 2-(β-hydroxyéthyl)-p-phénylènediamine, la 2-fluoro-p-phénylènediamine, la 2-isopropyl-p-phénylènediamine, la N-(β-hydroxypropyl)-p-phénylènediamine, la 2-hydroxyméthyl-p-phénylènediamine, la N,N-diméthyl-3-méthyl-p-phénylènediamine, la N,N-(éthyl,β-hydroxyéthyl)-p-phénylènediamine, la N-(β,γ-dihydroxypropyl)-p-phénylènediamine, la N-(4'-aminophényl)-p-phénylènediamine, la N-phényl-p-phénylènediamine, la 2-(β-hydroxyéthyloxy)-p-phénylènediamine, la 2-(β-acétylaminoéthyloxy)-p-phénylènediamine, la N-(β-méthoxyéthyl)-p-phénylènediamine, le 5,8-diaminobenzo-1,4-dioxanne, le N,N'-bis-(β-hydroxyéthyl)-N,N'-bis-(4'-amino-phényl)-1,3-diaminopropan-2-ol, la N,N'-bis-(β-hydroxyéthyl)-N,N'-bis-(4'-amino-phényl)éthylènediamine, la N,N'-bis-(4-aminophényl)tétraméthylènediamine, la N,N'-bis-(β-hydroxyéthyl)-N,N'-bis-(4-aminophényl)tétraméthylènediamine, la N,N'-bis-(4-méthylaminophényl)tétraméthylènediamine, la N,N'-bis-(éthyl)-N,N'-bis-(4'-amino-3'-méthylphényl)éthylènediamine, le 1,4-bis-(4'-aminophényl)diazacycloheptane, la N-(4'-aminophényl)-p-phénylènediamine et le 1,10-bis-(2',5'-diaminophényl)-1,4,7,10-tétraoxadécane ainsi que leurs sels physiologiquement acceptables.

8. Agents de teinture d'oxydation selon l'une quelconque des revendications 1 à 7, **caractérisés en ce que** le composant de développement est choisi parmi la p-phénylènediamine, la p-toluylènediamine, la 2,5-diméthyl-p-phénylènediamine, la 2,6-diméthyl-p-phénylènediamine, le 2-(2',5'-diaminophényl)éthanol, le 2-(2',5'-diaminophénoxy)éthanol, la N,N-bis-(2'-hydroxyéthyl)-p-phénylènediamine, la N,N'-bis-(2'-hydroxyéthyl)-pphénylènediamine, la 4-N,N-diméthylaminoaniline, le N,N'-bis-(2'-hydroxyéthyl)-N,N'-(4'-aminophényl)-1,3-diaminopropan-2-ol, le 1,3-bis-(2',5'-diaminophényl)propane, le 1,3-bis-(2',5'-diaminophénoxy)propane, le bis-(2,5-diaminophényl)méthane et la 4,4'-diaminodiphénylamine.

9. Agents de teinture d'oxydation selon l'une quelconque des revendications 1 à 8, **caractérisés en ce que** les composants de développement ainsi que les composants de couplage sont de préférence contenus en une quantité de 0,005 à 20% en poids par rapport à la totalité de l'agent de teinture d'oxydation.

10. Agents de teinture d'oxydation selon l'une quelconque des revendications 1 à 9, **caractérisés en ce qu'**ils contiennent au moins un dérivé indole et/ou indoline.

11. Agents de teinture d'oxydation selon l'une quelconque des revendications 1 à 10, **caractérisés en ce que** le support cosmétique est une crème, une émulsion, un gel, un shampooing ou un aérosol de mousse.

12. Procédé pour la teinture de fibres kératiniques, en particulier les cheveux, **caractérisé en ce qu'**on applique sur les fibres un agent de teinture d'oxydation selon l'une quelconque des revendications 1 à 11 avec un oxydant et/ou un catalyseur pour l'activation de l'oxydation et on rince ensuite, après un temps d'action, à nouveau avec de l'eau ou une composition tensioactive aqueuse.
